# FASCICULE DE BREVET EUROPEEN

(11) **EP 2 959 940 B1**
(45) Date de publication et mention de la délivrance du brevet: **14.12.2016**
(21) Numéro de dépôt: 15158146.9
(22) Date de dépôt: 09.03.2015
(51) Int. Cl.: A61N 1/375, A61N 1/372, A61B 5/00, A61N 1/05

(54) **Capsule implantable à fixation par vissage, notamment capsule autonome de stimulation cardiaque**
Implantierbare Kapsel mit Fixierung durch Verschraubung, insbesondere autonome Herzstimulationskapsel
Implantable capsule with attachment by screwing, in particular an autonomous cardiac stimulation capsule

(30) Priorité: 25.06.2014 FR 1455898
(43) Date de publication de la demande: 30.12.2015
(73) Titulaire: Sorin CRM SAS, 92140 Clamart (FR)
(72) Inventeur: Ollivier, Jean-François, 91190 Villiers le Bacle (FR)
(74) Mandataire: Dupuis-Latour, Dominique

(56) Documents cités:
- EP-A1- 2 537 555
- WO-A1-2012/088118
- US-A1- 2011 251 662
- US-A1- 2012 116 489
- US-A1- 2012 172 892
- US-A1- 2013 324 825

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif. L'invention concerne notamment, mais de manière non limitative, ceux de ces dispositifs qui se présentent sous forme d'une capsule autonome destinée à être implantée dans une cavité cardiaque (ventricule ou oreillette, à droite ou à gauche). Ces capsules sont dépourvues de toute liaison mécanique à un dispositif principal implanté (tel qu'un boitier de générateur d'impulsions de stimulation) ou non implanté (périphérique externe tel que programmateur ou dispositif de *monitoring* pour le suivi à distance du patient), et sont dénommées pour cette raison "capsules *leadless*", pour les distinguer des électrodes ou des capteurs disposés à l'extrémité distale d'une sonde (*lead*) conventionnelle, qui est parcourue sur toute sa longueur par un ou plusieurs conducteurs reliant par voie galvanique l'électrode ou le capteur à un générateur connecté à une extrémité opposée, proximale, de la sonde.

Le EP 2 394 695 A1 (Sorin CRM) décrit une capsule intracardiaque autonome, ainsi qu'une procédure permettant de l'implanter au site de détection/stimulation choisi et de la repositionner si nécessaire.

On notera toutefois, comme on le comprendra à la lecture de la description, que le caractère autonome de la capsule n'est pas intrinsèquement une caractéristique nécessaire de l'invention, et que cette dernière peut être aussi bien appliquée à des capsules montées de façon permanente à l'extrémité distale d'une sonde.

Une capsule implantable comprend un corps abritant les principaux éléments du dispositif (circuits électroniques, source d'énergie, électrodes de stimulations, etc.) et une embase solidaire du corps et supportant rigidement un moyen de fixation à la paroi.

Dans le cas des sondes cardiaques, deux types de fixation sont reconnus et traditionnellement employés : la fixation dite à barbes est la plus ancienne et encore marginalement utilisée, mais les sondes basées sur une vis de fixation ont supplanté les sondes à barbes et représentent actuellement la majorité du marché. Elles permettent une fixation généralement robuste et efficace. La vis est une vis hélicoïdale saillante prolongeant axialement le corps de la capsule et destinée à pénétrer dans le tissu cardiaque par vissage au site d'implantation, de la même manière que pour les sondes à vis conventionnelles.

Les WO 2012/088118 A1 et EP 2 537 555 A1 décrivent de telles structures de capsules *leadless* munies d'une vis hélicoïdale pour leur fixation à la paroi du coeur.

WO 2012/088118 A1 décrit les caractéristiques du préambule des revendications 1, 11 et 12.

Toutefois, la solidarisation entre le corps et le dispositif d'ancrage reste un point critique dans la mesure où un détachement accidentel du corps peut amener celui-ci à être libéré dans la cavité cardiaque puis transporté par le sang dans le système veineux ou artériel. Le risque de complication pour le patient est alors extrêmement élevé, ainsi que le risque de blessure du système cardiaque qui peut être engendré par toute zone saillante de l'implant comme par exemple une électrode pointue ou une arête saillante.

Davantage qu'un dispositif à sonde, un dispositif autonome subit quant à lui les contraintes et les mouvements engendrés par la paroi cardiaque, car il ne bénéficie pas de l'effort axial de maintien de la part du corps de sonde.

Pour remplir sa fonction d'ancrage permanent, le système de fixation doit donc être assujetti au corps de façon particulièrement sûre et à la fois pratique sur le plan industriel. La présente invention vise à proposer un dispositif autonome implantable qui réponde à ce besoin.

Plus précisément, l'invention propose une capsule implantable, notamment une capsule autonome de stimulation cardiaque, comprenant, de manière en elle-même connue un corps tubulaire pourvu à son extrémité distale d'un élément d'ancrage à vis hélicoïdale apte à pénétrer dans un tissu d'une paroi d'un organe d'un patient, le corps abritant un ensemble d'éléments fonctionnels de la capsule.

La capsule comprend un support annulaire solidaire du corps et généralement coaxial avec lui, ce support avoisinant une base de l'élément d'ancrage. De façon caractéristique de l'invention, le support annulaire comporte une série d'orifices s'étendant radialement, et la base de l'élément d'ancrage comporte une série de tiges ou tubes en saillie dans une direction radiale et engagés dans lesdits orifices et configurés pour assujettir l'élément d'ancrage audit support.

Selon diverses caractéristiques subsidiaires avantageuses :
- la capsule comprend en outre un anneau de diffusion de produit pharmaceutique, disposé à l'intérieur dudit support, et il est prévu des orifices en nombre supérieur au nombre de tiges ou tubes, de manière à favoriser la circulation du produit vers l'extérieur du support ;
- les tiges ou tubes sont rapportés sur la base de l'élément d'ancrage par tir laser, ou fixés à la matière du support au niveau des orifices par tir laser ;
- la capsule comprend un cordon de soudure fixant le support au corps ;
- le corps et le support sont en acier inoxydable, tandis que l'élément d'ancrage et les tiges ou tubes sont en alliage de titane ;
- les orifices sont régulièrement répartis angulairement à la périphérie du support, les tiges ou tubes pouvant notamment être situés dans deux orifices adjacents, les autres orifices étant libres ;
- les orifices ont une section correspondant à celle des tiges ou tubes ; et
- les orifices comprennent des encoches en forme générale de L s'étendant à partir d'une face libre du support, permettant un montage de type baïonnette de l'élément d'ancrage pourvu de tiges ou tubes dans le support.

L'invention a également pour objet un procédé d'assemblage d'une capsule implantable telle que ci-dessus, comprenant les étapes suivantes :
a) fixer au corps, par soudage dans une position coaxiale avec ledit corps, un support annulaire possédant une série d'orifices s'étendant radialement,
b) engager une base de l'élément d'ancrage à l'intérieur du support,
c) engager dans les orifices du support une série de tiges ou tubes,
d) souder les tiges ou tubes sur la base de l'élément d'ancrage, par tir laser via l'intérieur du support, et
e) souder les tiges ou tubes à la matière du support dans leurs orifices respectifs, par tir laser extérieurement au support.

Dans une variante, le procédé comprend les étapes suivantes :
a) fixer au corps, par soudage dans une position coaxiale avec le corps, un support annulaire possédant une série d'orifices s'étendant radialement et formés par des encoches en forme générale de L,
b) souder une série de tiges ou tubes sur une base de l'élément d'ancrage, par tir laser, les tiges ou tubes s'étendant radialement,
c) pré-assembler l'élément d'ancrage, par sa base, au support, par montage de type baïonnette mettant en jeu les tiges ou tubes et les encoches, et
d) souder les tiges ou tubes à la matière du support dans les encoches, par tir laser extérieurement au support.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 est une vue d'ensemble en perspective d'une capsule implantable selon l'invention.
La Figure 2 est une vue en perspective et en demi-coupe d'une région distale de la capsule de la Figure 1.
La Figure 3 est une vue en perspective d'un support de vis d'ancrage de la capsule des Figures 1 et 2.
La Figure 4 est une vue en perspective d'un détail du support de la Figure 3.
La Figure 5 est une vue de profil du détail de la Figure 4.
La Figure 6 est une vue en perspective et en demi-coupe d'une région distale d'une capsule incorporant une variante de réalisation,
La Figure 7 est une vue en perspective, selon une autre orientation, de cette même région distale.
La Figure 8 est une vue en perspective d'un ensemble vis-support selon un autre aspect d'une capsule implantable, la vis étant visible en transparence.
La Figure 9 est une vue en perspective de l'ensemble vis-support de la Figure 8.
La Figure 10 est une vue partielle en perspective d'une variante d'aménagement du support pour la fixation de la vis sur le support.

On va maintenant décrire un exemple de réalisation du dispositif de l'invention.

On va maintenant décrire des exemples de réalisation d'une capsule implantable.

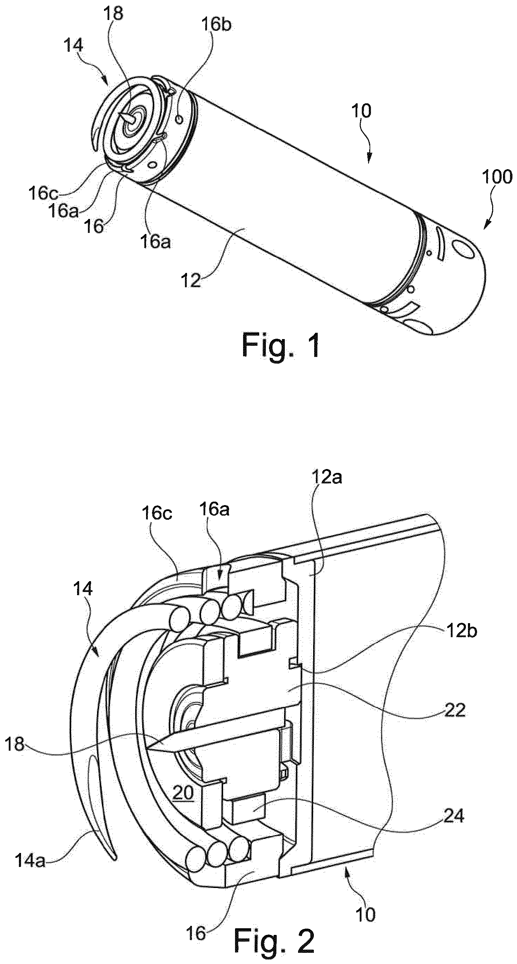

En référence tout d'abord à la Figure 1, on a représenté une capsule implantable 10, ici une capsule autonome de stimulation cardiaque, comprenant un corps de capsule 12 et un organe d'ancrage de type vis hélicoïdale 14.

La vis 14 est formée par un fil enroulé en hélice à pas de vissage à droite et est montée sur un support de vis 16 intégrant des aménagements assurant l'irréversibilité de l'ancrage comme on va le voir dans la suite.

La vis 14 est rendue définitivement solidaire de son support 16 par exemple par soudage laser selon les points de soudure répartis. L'ensemble est ensuite solidarisé définitivement au corps de la capsule 12 par un cordon de soudure laser.

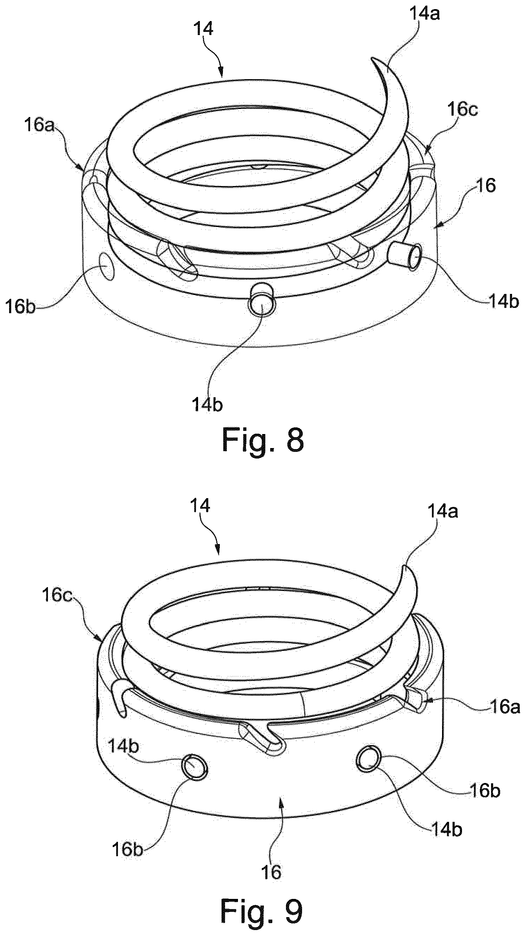

On notera que les matériaux de la vis 14 et de son support 16 peuvent être différents, par exemple un couple platine/iridium 90/10 pour la vis 14 et du titane pour le support 16. Il est en effet possible, comme on le verra plus bas en référence au mode de réalisation illustré Figures 8 et 9, de mettre en oeuvre une méthode d'assemblage efficace de ces deux éléments même s'ils sont réalisés dans des matériaux hétérogènes.

Les différentes pièces sont par exemple réalisées en alliages métalliques biocompatibles tels qu'un acier inoxydable ou un alliage de titane, de préférence un alliage de titane biocompatible pour le corps de capsule 12 et le support 16.

Les aménagements pour l'irréversibilité de la fixation comprennent des encoches ou évidements 16a formés dans le support 16.

L'ensemble de fixation constitué des pièces 14, 16 présente un diamètre de préférence égal à celui du corps de l'implant, typiquement de 6 à 7 mm, et une longueur axiale légèrement inférieure, typiquement entre 4 et 6 mm. La vis 14 délimite un espace libre occupé par un autre sous-ensemble du dispositif, ici un sous-ensemble d'électrodes de stimulation. Le système de fixation est conçu pour fixer l'implant de façon stable et durable dans le temps grâce à la vis hélicoïdale 14 formant un ressort à spires évolutives et se terminant par une pointe apte à perforer l'endothélium et à pénétrer dans les tissus musculaires, de façon à plaquer la paroi cardiaque sur la face frontale généralement annulaire du support 16, sensiblement à la même position (en direction axiale) que la surface d'appui interne du système d'électrode.

Plus en détail, le support de vis 16 présente sur ladite face frontale 16c une série d'évidements 16a qui réalisent la fonction d'irréversibilité une fois en contact avec l'endothélium. Dans cette position, le système de fixation est indémontable : sous l'action de la vis 14 qui agit comme un ressort de traction réalisant un effort de retenue axial, la paroi cardiaque est plaquée contre la face 16c du support de vis 16 et s'ancre localement dans les évidements (ici au nombre de six, régulièrement répartis) par l'effet ressort précité.

Comme on va le voir plus en détail dans la suite, les six évidements définissent autant d'arêtes vives, mais peu profondes, dans leur région (en direction circonférentielle) opposée au sens de vissage de l'hélice et réalisent alors six points d'ancrage ponctuels et répartis sur l'endothélium (espacement mutuel de 60° dans cet exemple), qui assurent une irréversibilité du vissage réalisé pour ancrer la capsule.

Maintenant plus en détail et en référence à la Figure 2, on observe un sous-ensemble de stimulation constitué ici par deux électrodes 18 et 20 maintenues par un support d'électrodes 22 qui supporte également à sa périphérie un anneau 24 de diffusion d'un produit stéroïde.

Le support 22 est positionné coaxialement au support de vis 16, à l'intérieur de celui-ci, et fixé par collage ou tout autre moyen approprié sur une paroi 12a fermant l'espace intérieur du corps de capsule 12 à son extrémité distale côté vis, le support étant localisé précisément grâce à un épaulement circulaire 12b prévu sur la face extérieure de ladite paroi. L'électrode de stimulation centrale en pointe 18 est fixée (par sertissage, collage, etc.) au centre du support 22 et connectée électriquement à l'électronique interne de la capsule logée dans le corps 12, par l'intermédiaire d'une micro-traversée. La deuxième électrode 20 présente ici la forme d'une rondelle positionnée et fixée, par exemple par collage, sur la face externe du support 22 et est connectée également par le biais d'une micro-traversée à l'électronique interne.

L'anneau 24 est imprégné d'un produit stéroïde tel que la dexaméthasone et est positionné sous l'électrode 20. Le produit stéroïde peut diffuser via l'espace subsistant entre la vis 14 et la deuxième électrode 20 et entrer en contact avec la paroi cardiaque, ce qui permet de réduire l'inflammation tissulaire durant les premières semaines qui suivent l'implantation. L'électronique associée aux électrodes permet de mettre en oeuvre, dans le cas d'une stimulation cardiaque, des fonctions de détection et de stimulation, la structure décrite garantissant un contact fiable et permanent entre les électrodes 18, 20 et les tissus.

La vis en hélice 14 est ici constituée d'un fil métallique d'un diamètre de l'ordre de 0,5 mm, avec un diamètre d'enroulement typiquement 5 mm environ et de préférence identique à celui du corps 12 du dispositif. La vis comprend une base plane suivie par deux spires jointives et une spire finale s'étendant par exemple sur environ 1,5 tour avec un espace interspire du même ordre de grandeur que le diamètre du fil. L'extrémité libre de la vis 14 est affinée, dans le cas d'espèce par deux usinages dans des plans mutuellement orthogonaux, créant ainsi une pointe 14a perforante mais non tranchante. Le but de cette pointe est de permettre de traverser facilement l'endothélium puis de pénétrer dans le muscle cardiaque tout en créant un minimum de lésions tissulaires. La vis 14 est ici réalisée en acier inoxydable 316L implantable et biocompatible ou toute autre matière équivalente, qui délivre une raideur d'environ 0,1 N/mm (raideur linéaire du ressort, mesurée par traction ou compression à l'aide d'un dynamomètre sur une course de 1 mm).

Ceci donne à la vis une souplesse axiale qui lui confère un effet ressort, opérant un effort de traction assurant un contact ferme entre le support 16 et la paroi cardiaque. Ainsi lors de la pénétration de la vis 14 dans le muscle, la vis se déforme axialement jusqu'au contact du bord libre 16c du support 16 avec l'endothélium. L'effet ressort de la vis va alors comprimer axialement l'endothélium et le muscle entre les spires et créer un effet de coincement. De plus, la proximité immédiate des spires et leur effet de traction sur l'endothélium forcent l'entrée de ce dernier dans les encoches d'antidévissage. Au cours de ce mouvement, l'électrode de stimulation en pointe 18 perfore l'endothélium et vient ainsi en contact avec les cellules excitables de la paroi cardiaque.

D'autres configurations de spires sont bien entendu possibles, mais il est avantageux de prévoir un espacement entre spires qui augmente entre la base de la vis (où comme on l'a vu l'espacement peut être nul) et la pointe 14a de la vis. Ceci permet de favoriser l'effort de traction axial appliquant le support 16 contre la paroi cardiaque.

On comprend par ailleurs que l'électrode 18 contribue à la fixation de la capsule tout en assurant contact fiable et continu avec les tissus comprimés grâce à l'effort axial de la vis 14 en direction du support 16.

Une telle configuration est particulièrement adaptée pour une stimulation basse énergie, avec une longueur d'aiguille de l'ordre de 1,2 mm et un diamètre de l'ordre de 0,4 mm, soit une surface active de l'ordre de 1 mm².

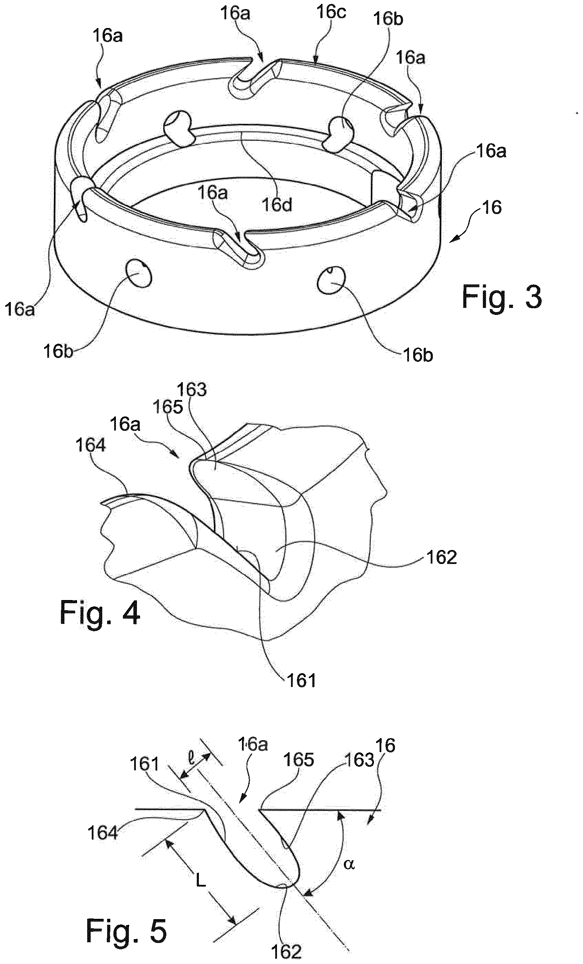

En référence tout particulièrement aux Figures 3 à 5, le support 16 de la vis 14 présente comme on l'a indiqué une face ou bord d'appui 16c, de préférence de profil légèrement arrondi, à partir duquel sont formés, en direction oblique, six évidements 16a permettant de réaliser six arêtes saillantes 165 très localisées et très ponctuelles (voir notamment Figures 4 et 5) assurant l'irréversibilité du vissage de la capsule réalisé comme décrit précédemment. La perforation localisée de l'endothélium est obtenue par les arêtes saillantes 165, les autres arêtes qui forment les limites de l'encoche étant volontairement très arrondies afin de ne pas propager ni agrandir le percement de l'endothélium lors des efforts de dévissage. La surface d'appui supportant les contraintes de dévissages étant maximisée, les risques de déchirements de l'endothélium sont minimisés. Préférentiellement mais non limitativement, la surface relative occupée par les évidements de la face d'appui 16c représente de 20 à 40 % de cette surface, et plus préférentiellement autour de 30 %, ce qui permet de limiter l'effet traumatique des arêtes saillantes 165 en limitant la pénétration des tissus dans les évidements 16a, tout en assurant un bon accrochage desdites arêtes 165 dans l'endothélium, et donc l'efficacité de la fonction antidévissage.

Les dimensions des évidements peuvent varier. Par exemple et en référence particulièrement à la Figure 5, leur largeur f peut être de l'ordre de 0,3 à 0,8 mm, leur profondeur ou longueur L peut être comprise entre 0,5 et 1 mm, et leur inclinaison α peut être comprise entre 20 et 60°, et de préférence voisine de 45°.

Dans une variante de mise en oeuvre avantageuse, les évidements 16a définissant les arêtes saillantes 165 sont réalisés dans une pièce biodégradable rapportée sur le support 16. La matière utilisée pour réaliser cette pièce rapportée peut être notamment un biopolymère bioabsorbable tel qu'un polylactide (PLA), un polyglycolide (PGA) ou un polylactide-co-glycolide (PLGA), ou toute autre matière équivalente, implantable et résorbable dans l'organisme. La fonction anti-dévissage sera alors assurée pendant une période prédéfinie, par exemple de 3 à 12 mois, pendant laquelle la fibrose recouvrira progressivement l'embase de la capsule. Puis, à long terme, il deviendra plus facile de retirer la capsule une fois la fonction anti-dévissage disparue.

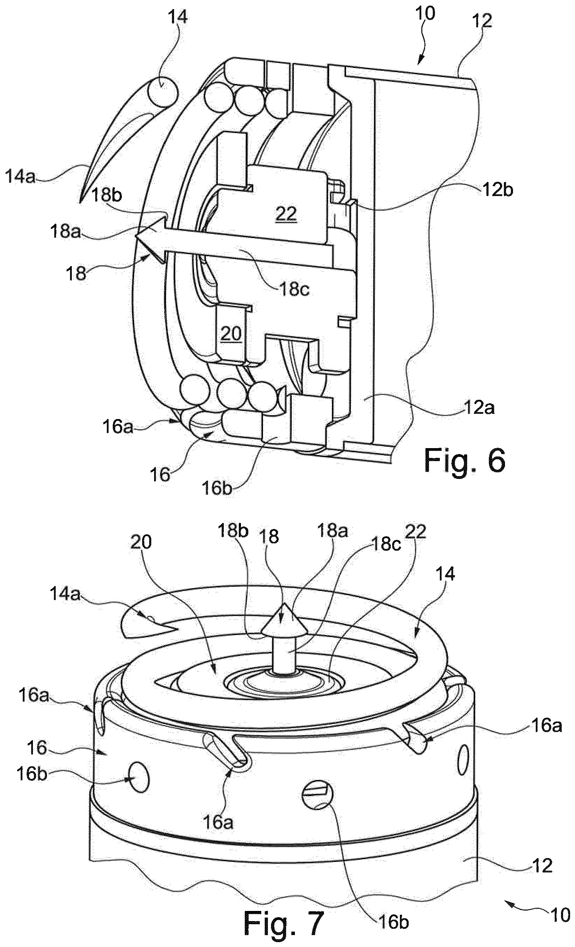

En référence maintenant aux Figures 6 et 7, on va décrire une variante de réalisation de l'électrode en pointe 18. Celle-ci présente dans ce cas une tête élargie 18a, de préférence de forme conique, rejoignant à sa base, par un épaulement annulaire rentrant 18b, une tige plus étroite 18c. L'épaulement 18b offre une surface qui renforce mécaniquement la stabilité de la capsule sur la paroi cardiaque, en participant à la retenue des tissus comprimés par l'effort axial de la vis 14.

Dans le présent exemple, l'épaulement 18b présente une largeur annulaire de l'ordre de 0,2 mm. Cette valeur peut varier selon les besoins et les dimensions de la capsule, typiquement entre 0.05 et 1 mm.

L'angle du cône est quant à lui compris entre environ 30 et 60°, et typiquement voisin de 50°.

On va maintenant décrire une solution d'assemblage permanent de la vis 14 sur son support 16, cette solution pouvant être mise en oeuvre indépendamment des caractéristiques d'antidévissage et d'accrochage à la paroi cardiaque telles que détaillées dans ce qui précède.

Le problème que résout cette solution est de pouvoir assembler une vis 14 en acier inox ou autre matériau non soudable directement sur le boitier en titane de la capsule.

Ainsi, en référence aux Figures 8 et 9, le support 16 présente une configuration comprenant un ensemble d'orifices traversants 16b, ayant une section correspondant approximativement à celle des tiges ou tubes et par exemple circulaire, formés dans la paroi du support 16, ces orifices recevant des tiges ou tubes homologues 14b formés à la périphérie de l'embase de la vis 14, dans des emplacements angulaires correspondants. Dans le présent exemple, on a prévu deux orifices traversants 16b, espacés par exemple de 60°, et deux tiges ou tubes correspondants 14b. Plus précisément, on glisse sous léger serrage d'abord la vis 14 dans son support 16 jusqu'en butée axiale contre un épaulement formé dans l'alésage central du support. Plusieurs orifices traversants radiaux 16b sont aménagés dans le support 16, qui coïncident avec la partie de la vis à spire jointive. Une tige ou un tube 14b du même matériau que la vis, ou en un matériau soudable par laser au matériau de la vis, est alors inséré dans chacun de ces trous latéraux. Dans le cas (préféré) d'un tube, l'alésage des tubes débouche alors sur les spires de la vis. Un tir laser à travers ces trous permet alors une soudure directe tube/vis assurant les conditions requises d'une bonne soudure laser, à savoir : i) compatibilité des matériaux, ii) contact direct et iii) accès visuel pour le tir et le contrôle de qualité.

Cette soudure effectuée, la vis se trouve bloquée en translation et rotation dans son support, par ancrage mécanique et sans soudure laser directe, ce qui laisse une grande liberté dans la combinaison de matériaux support/vis. Les autres avantages sensibles de cette solution sont le faible encombrement et la très faible complexité des usinages à réaliser.

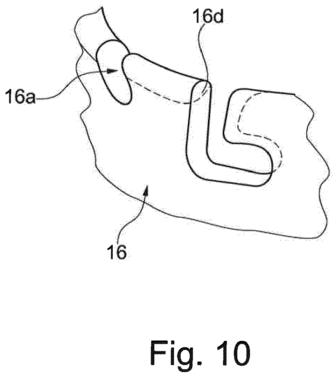

Selon une variante de réalisation illustrée sur la Figure 10 des dessins, on peut prévoir entre la vis 14 et son support 16 un montage de type baïonnette, la vis 14 comportant alors au niveau de sa base deux tiges ou tubes tels que 14b, de préférence diamétralement opposés, et le support 16 comportant dans deux régions diamétralement opposées des encoches 16d en forme générale de L s'étendant à partir de la face libre du support 16 et permettant, avant le soudage par tir laser, le montage de la vis 14 par translation axiale pour l'engagement des tiges ou tubes dans les encoches, suivie d'une rotation pour le verrouillage des tiges ou tubes au fond des encoches. Il peut alors exister, outre ces deux encoches, d'autres encoches 16d et/ou d'autres orifices circulaires 16b.

Dans le premier cas décrit plus haut, le procédé d'assemblage de la capsule comprend de préférence les étapes suivantes :
a) on fixe le support 16 au corps, par soudage,
b) on engage la base de l'élément 14 à l'intérieur dudit support,
c) on engage dans les orifices 16b les tiges ou tubes 14b,
d) on soude les tiges ou tubes sur la base de l'élément 14, par tir laser via l'intérieur du support, et
e) on soude les tiges ou tubes dans leurs orifices respectifs, par tir laser extérieurement au support.

On notera que le "soudage" de l'étape d) ne doit pas être entendu au sens étroit d'une soudure mécanique avec fusion de la matière de deux pièces distinctes, mais d'une opération destinée à effondrer la matière de la tige ou du tube à l'intérieur du logement supportant les reprises mécaniques et renforcer la fonction atraumatique par suppression des formes saillantes.

Dans le cas de la variante de la Figure 10, le procédé comprend de préférence les étapes suivantes :
a) on fixe le support 16 pourvu des encoches 16d au corps, par soudage,
b) on soude les tiges ou tubes sur la base de l'élément 14, par tir laser,
c) on pré-assemble l'élément d'ancrage par sa base dans le support, par montage de type baïonnette mettant en jeu lesdits tiges ou tubes et lesdites encoches, et
d) on soude les tiges ou tubes dans les encoches, par tir laser extérieurement au support.

Avantageusement, on prévoit des orifices 16b ou 16d en nombre supérieur au nombre de tiges ou tubes 14b de la vis 14. De la sorte, les orifices 16b ou 16d restant libres permettent de faciliter la diffusion du produit stéroïde délivré par l'anneau 24 radialement en direction de la paroi cardiaque.

Selon une autre variante, on peut prévoir que les encoches 16d prévues pour un tel montage de type baïonnette soient formées de façon à participer à une fonction d'antidévissage comme décrit précédemment.

Les différents éléments et parties décrits ci-dessus peuvent être réalisés par usinage ou autre mise en forme selon des techniques conventionnelles.

Par ailleurs, on peut prévoir comme décrit plus haut que le support 16 entoure la base de l'élément d'ancrage, mais alternativement que la base de l'élément d'ancrage entoure le support, auquel cas l'homme du métier saura faire les adaptations nécessaires, notamment quant au positionnement des tiges ou tubes 14b assurant la fixation entre l'élément d'ancrage et le support.

La capsule peut être posée par le praticien selon la technique décrite notamment dans le EP 2 394 695 A1, et extraite également selon une technique connue, la partie proximale 12 du corps de la capsule étant aménagée de façon adaptée.

L'invention ne se limite nullement à la fixation d'une capsule de stimulation autonome dans une paroi cardiaque, mais peut être mise en oeuvre pour d'autres systèmes implantables, qu'ils soient autonomes ou en bout de sonde, du corps humain. En fonction de la nature de la paroi de fixation visée, les longueurs d'hélice et le cas échéant d'électrode peuvent être facilement adaptées par l'homme du métier, sans pour autant dégrader les performances de fixation et d'irréversibilité.

## Revendications

1. Capsule implantable (10), notamment capsule autonome de stimulation cardiaque, comprenant un corps tubulaire (12) pourvu à son extrémité distale d'un élément d'ancrage à vis hélicoïdale (14) apte à pénétrer dans un tissu d'une paroi d'un organe d'un patient, le corps abritant un ensemble d'éléments fonctionnels de la capsule,
un support annulaire (16) solidaire du corps (12) et généralement coaxial avec lui, ce support avoisinant une base de l'élément d'ancrage
**caractérisée en ce que** le support annulaire (16) comporte une série d'orifices (16b ; 16d)
s'étendant radialement,
et **en ce que** la base de l'élément d'ancrage comporte une série de tiges ou tubes (14b) en saillie dans une direction radiale et engagés dans lesdits orifices et configurés pour assujettir l'élément d'ancrage audit support (16).

2. Capsule selon la revendication 1, **caractérisée en ce qu'**elle comprend en outre un anneau (24) de diffusion de produit pharmaceutique, disposé à l'intérieur dudit support (16), et **en ce qu'**il est prévu des orifices (16b ; 16d) en nombre supérieur au nombre de tiges ou tubes, de manière à favoriser la circulation du produit vers l'extérieur du support.

3. Capsule selon la revendication 1, **caractérisée en ce que** les tiges ou tubes (14b) sont rapportés sur la base de l'élément d'ancrage (14) par tir laser.

4. Capsule selon la revendication 1, **caractérisée en ce que** les tiges ou tubes (14b) sont fixés à la matière dudit support (16) au niveau des orifices (16b ; 16d) par tir laser.

5. Capsule selon la revendication 1, **caractérisée en ce qu'**elle comprend un cordon de soudure fixant le support (16) au corps (12).

6. Capsule selon la revendication 1, **caractérisée en ce que** le corps (12) et le support (16) sont en acier inoxydable, tandis que l'élément d'ancrage (14) et les tiges ou tubes (14b) sont en alliage de titane.

7. Capsule selon la revendication 1, **caractérisée en ce que** les orifices (16b, 16d) sont régulièrement répartis angulairement à la périphérie du support.

8. Capsule selon la revendication 7, **caractérisée en ce que** les tiges ou tubes (14b) sont situés dans deux orifices (16b) adjacents, les autres orifices étant libres.

9. Capsule selon la revendication 1, **caractérisé en ce que** les orifices (16b) ont une section correspondant à celle des tiges ou tubes (14b).

10. Capsule selon la revendication 1, **caractérisé en ce que** les orifices comprennent des encoches (16d) en forme générale de L s'étendant à partir d'une face libre du support, permettant un montage de type baïonnette de l'élément d'ancrage pourvu de tiges ou tubes dans le support (16).

11. Procédé d'assemblage d'une capsule implantable (10) telle qu'une capsule autonome de stimulation cardiaque, ladite capsule comprenant un corps tubulaire (12) pourvu à son extrémité distale d'un élément d'ancrage à vis hélicoïdale (14) apte à pénétrer dans un tissu d'une paroi d'un organe d'un patient, le corps abritant un ensemble d'éléments fonctionnels de la capsule, le procédé étant **caractérisé par** les étapes suivantes :
a) fixer au corps (12), par soudage dans une position coaxiale avec ledit corps, un support annulaire (16) possédant une série d'orifices (16b) s'étendant radialement,
b) engager une base de l'élément d'ancrage à l'intérieur dudit support,
c) engager dans lesdits orifices (16b) du support une série de tiges ou tubes (14b),
d) souder les tiges ou tubes sur la base de l'élément d'ancrage, par tir laser via l'intérieur du support, et
e) souder les tiges ou tubes à la matière du support dans leurs orifices respectifs, par tir laser extérieurement au support.

12. Procédé d'assemblage d'une capsule implantable (10) telle qu'une capsule autonome de stimulation cardiaque, ladite capsule comprenant un corps tubulaire (12) pourvu à son extrémité distale d'un élément d'ancrage à vis hélicoïdale (14) apte à pénétrer dans un tissu d'une paroi d'un organe d'un patient, le corps abritant un ensemble d'éléments fonctionnels de la capsule, le procédé étant **caractérisé par** les étapes suivantes :
a) fixer au corps (12), par soudage dans une position coaxiale avec ledit corps, un support annulaire (16) possédant une série d'orifices (16d) s'étendant radialement et formés par des encoches en forme générale de L,
b) souder une série de tiges ou tubes (14b) sur une base de l'élément d'ancrage (14), par tir laser, lesdits tiges ou tubes s'étendant radialement,
c) pré-assembler l'élément d'ancrage, par sa base, au support, par montage de type baïonnette mettant en jeu les tiges ou tubes et les encoches, et
d) souder les tiges ou tubes à la matière du support dans les encoches, par tir laser extérieurement au support.

13. Procédé selon la revendication 11 ou 12, **caractérisé en ce que** le corps (12) et le support (16) sont en acier inoxydable, tandis que l'élément d'ancrage (14) et les tiges ou tubes (14b) sont en alliage de titane.

## Patentansprüche

1. Implantierbare Kapsel (10), insbesondere autonome Herzstimulationskapsel, umfassend einen rohrförmigen Körper (12), der an seinem distalen Ende mit einem Verankerungselement mit schraubenförmiger Schnecke (14) versehen ist, das geeignet ist, in ein Gewebe einer Wand eines Organs eines Patienten einzudringen, wobei in dem Körper eine Gruppe von Funktionselementen der Kapsel untergebracht ist,
einen ringförmigen Träger (16), der mit dem Körper (12) fest verbunden ist und im Allgemeinen koaxial mit ihm ist, wobei dieser Träger an eine Basis des Verankerungselements grenzt, **dadurch gekennzeichnet, dass** der ringförmige Träger (16) eine Reihe von Öffnungen (16b; 16d) aufweist, die sich radial erstrecken,
und dass die Basis des Verankerungselements eine Reihe von Stangen oder Rohren (14b) aufweist, die in einer radialen Richtung vorstehen und in den Öffnungen in Eingriff stehen und konfiguriert sind, um das Verankerungssystem an dem Träger (16) zu befestigen.

2. Kapsel nach Anspruch 1, **dadurch gekennzeichnet, dass** sie ferner einen Ring (24) zur Verteilung des Arzneimittels aufweist, der im Inneren des Trägers (16) angeordnet ist, und dass Öffnungen (16b; 16d) in einer größeren Anzahl als die Anzahl an Stangen oder Rohren derart vorgesehen sind, um das Zirkulieren des Produktes zur Außenseite des Trägers zu begünstigen.

3. Kapsel nach Anspruch 1, **dadurch gekennzeichnet, dass** die Stangen oder Rohre (14b) durch Laserschuss auf der Basis des Verankerungselements (14) angebracht sind.

4. Kapsel nach Anspruch 1, **dadurch gekennzeichnet, dass** die Stangen oder Rohre (14b) durch Laserschuss an dem Material des Trägers (16) auf Ebene der Öffnungen (16b; 16d) befestigt sind.

5. Kapsel nach Anspruch 1, **dadurch gekennzeichnet, dass** sie eine Schweißnaht aufweist, die den Träger (16) an dem Körper (12) befestigt.

6. Kapsel nach Anspruch 1, **dadurch gekennzeichnet, dass** der Körper (12) und der Träger (16) aus Edelstahl sind, während das Verankerungselement (14) und die Stangen oder Rohre (14b) aus Titanlegierung sind.

7. Kapsel nach Anspruch 1, **dadurch gekennzeichnet, dass** die Öffnungen (16b, 16d) am Umfang des Trägers gleichmäßig winkelmäßig verteilt sind.

8. Kapsel nach Anspruch 7, **dadurch gekennzeichnet, dass** die Stangen oder Rohre (14b) in den zwei angrenzenden Öffnungen (16b) angeordnet sind, während die anderen Öffnungen frei sind.

9. Kapsel nach Anspruch 1, **dadurch gekennzeichnet, dass** die Öffnungen (16b) einen Querschnitt aufweisen, der jenem der Stangen oder Rohre (14b) entspricht.

10. Kapsel nach Anspruch 1, **dadurch gekennzeichnet, dass** die Öffnungen allgemein L-förmige Kerben (16d) aufweisen, die sich ausgehend von einer freien Fläche des Trägers erstrecken, die eine Montage von der Art einer Bajonettmontage des Verankerungselements, das mit Stangen oder Rohren versehen ist, in dem Träger (16) ermöglichen.

11. Verfahren zum Zusammenbauen einer implantierbaren Kapsel (10), wie eine autonome Herzstimulationskapsel, wobei die Kapsel einen rohrförmigen Körper (12) aufweist, der an seinem distalen Ende mit einem Verankerungselement mit schraubenförmiger Schnecke (14) versehen ist, das geeignet ist, in ein Gewebe einer Wand eines Organs eines Patienten einzudringen, wobei in dem Körper eine Gruppe von Funktionselementen der Kapsel untergebracht ist, wobei das Verfahren durch die folgenden Schritte gekennzeichnet ist:
a) an dem Körper (12) durch Schweißen in einer Position, die koaxial mit dem Körper ist, einen ringförmigen Träger (16) befestigen, der eine Reihe von Öffnungen (16b) aufweist, die sich radial erstrecken,
b) eine Basis des Verankerungselements im Inneren des Trägers in Eingriff bringen,
c) in den Öffnungen (16b) des Trägers eine Reihe von Stangen oder Rohren (14b) in Eingriff bringen,
b) die Stangen oder Rohre durch Laserschuss über das Innere des Trägers auf die Basis des Verankerungselements schweißen und
e) die Stangen oder Rohre durch Laserschuss außen an dem Träger an das Material des Trägers in ihren jeweiligen Öffnungen anschweißen.

12. Verfahren zum Zusammenbauen einer implantierbaren Kapsel (10), wie eine autonome Herzstimulationskapsel, wobei die Kapsel einen rohrförmigen Körper (12) aufweist, der an seinem distalen Ende mit einem Verankerungselement mit schraubenförmiger Schnecke (14) versehen ist, das geeignet ist, in ein Gewebe einer Wand eines Organs eines Patienten einzudringen, wobei in dem Körper eine Gruppe von Funktionselementen der Kapsel untergebracht ist, wobei das Verfahren durch die folgenden Schritte gekennzeichnet ist:
a) an dem Körper (12) durch Schweißen in einer Position, die koaxial mit dem Körper ist, einen ringförmigen Träger (16) befestigen, der eine Reihe von Öffnungen (16d) aufweist, die sich radial erstrecken und durch allgemein L-förmige Kerben gebildet sind,
b) eine Reihe von Stangen oder Rohren (14b) durch Laserschuss auf die Basis des Verankerungselements (14) schweißen, wobei sich die Stangen oder Rohre radial erstrecken,
c) das Verankerungselement durch Montage von der Art einer Bajonettmontage durch Einsetzen der Stangen oder Rohre und der Kerben mit seiner Basis an den Träger vormontieren und
d) die Stangen oder Rohre durch Laserschuss außen an dem Träger an das Material des Trägers in die Kerben schweißen.

13. Verfahren nach Anspruch 11 oder 12, **dadurch gekennzeichnet, dass** der Körper (12) und der Träger (16) aus Edelstahl sind, während das Verankerungselement (14) und die Stangen oder Rohre (14b) aus Titanlegierung sind.

## Claims

1. An implantable capsule (10), in particular an autonomous capsule for cardiac stimulation, comprising a tubular body (12) provided at its distal end with an anchoring element (14) having an helical screw and adapted to penetrate a tissue of a patient's organ wall, the body housing a set of functional elements of the capsule, an annular support (16) integral with the body (12) and generally coaxial with the latter, said support being close to a base of the anchoring element **characterized in that** the annular support (16) includes a series of orifices (16b; 16d) extending radially, and **in that** the base of the anchoring element includes a series of rods or tubes (14b) protruding in a radial direction and engaged in said orifices and configured to secure the anchoring element to said support (16).

2. The capsule according to claim 1, **characterized in that** it further comprises a pharmaceutical product distribution ring (24), arranged inside said support (16), and **in that** orifices (16b; 16d) are provided in a number higher than the number of rods or tubes, so as to favour the circulation of the product towards the outside of the support.

3. The capsule according to claim 1, **characterized in that** the rods or tubes (14b) are added on the base of the anchoring element (14) by laser shooting.

4. The capsule according to claim 1, **characterized in that** the rods or tubes (14b) are fastened to the material of said support (16) at the orifices (16b; 16d) by laser shooting.

5. The capsule according to claim 1, **characterized in that** it comprises a welding bead fastening the support (16) to the body (12).

6. The capsule according to claim 1, **characterized in that** the body (12) and the support (16) are made of stainless steel, whereas the anchoring element (14) and the rods or tubes (14b) are made of a titanium alloy.

7. The capsule according to claim 1, **characterized in that** the orifices (16b, 16d) are regularly angularly distributed at the periphery of the support.

8. The capsule according to claim 7, **characterized in that** the rods or the tubes (14b) are located in two adjacent orifices (16b), the other orifices being free.

9. The capsule according to claim 1, **characterized in that** the orifices (16b) have a section corresponding to that of the rods or tubes (14b).

10. The capsule according to claim 1, **characterized in that** the orifices comprise generally L-shaped notches (16d) extending from a free face of the support, allowing a bayonet-type mounting of the anchoring element provided with rods or tubes into the support (16).

11. A method of assembling an implantable capsule (10) such as an autonomous capsule for cardiac stimulation, said capsule comprising a tubular body (12) provided at its distal end with an anchoring element (14) having an helical screw and adapted to penetrate a tissue of a patient's organ wall, the body housing a set of functional elements of the capsule, the method being **characterized by** the following steps:
a) fastening to the body (12), by welding in a position coaxial with said body, an annular support (16) having a series of orifices (16b) extending radially,
b) engaging a base of the anchoring element inside said support,
c) engaging into said orifices (16b) of the support a series of rods or tubs (14b),
d) welding the rods or tubes to the base of the anchoring element, by laser shooting, from the inside of the support, and
e) welding the rods or tubes to the material of the support, inside their respective orifices, by laser shooting from the outside of the support.

12. A method of assembling an implantable capsule (10) such as an autonomous capsule for cardiac stimulation, said capsule comprising a tubular body (12) with an anchoring element (14) having an helical screw and adapted to penetrate a tissue of a patient's organ wall, the body housing a set of functional elements of the capsule, the method being **characterized by** the following steps:
a) fastening to the body (12), by welding in a position coaxial with said body, an annular support (16) having a series of orifices (16b) extending radially and formed by generally L-shaped notches,
b) welding a series of rods or tubes (14b) to a base of the anchoring element (14), by laser shooting, said rods or tubes extending radially,
c) pre-assembling the anchoring element, by its base, to the support, by a bayonet-type mounting implementing the rods or tubes and the notches, and
d) welding the rods or tubes to the material of the support, inside the notches, by laser shooting from the outside of the support.

13. The method according to claim 11 or 12, **characterized in that** the body (12) and the support (16) are made of stainless steel, whereas the anchoring element (14) and the rods or tubes (14b) are made of a titanium alloy.
